# EUROPÄISCHE PATENTSCHRIFT

(11) **EP 2 912 447 B1**
(45) Veröffentlichungstag und Bekanntmachung des Hinweises auf die Patenterteilung: **21.02.2018**
(21) Anmeldenummer: 13771132.1
(22) Anmeldetag: 30.09.2013
(51) Int. Cl.: G01N 27/417, G01N 27/419

(54) **VERFAHREN UND VORRICHTUNG ZUR DIAGNOSE DES LUFTREFERENZKANALS EINER BREITBAND-LAMBDASONDE**
METHOD AND APPARATUS FOR DIAGNOSING THE AIR REFERENCE CHANNEL OF A BROAD BAND LAMBDA PROBE
PROCEDE ET APPAREIL POUR FAIRE UN DIAGNOSTIC DU CANAL DE REFERENCE D'AIR D'UNE SONDE LAMBDA A BANDE LARGE

(30) Priorität: 23.10.2012 DE 102012219282
(43) Veröffentlichungstag der Anmeldung: 02.09.2015
(73) Patentinhaber: Robert Bosch GmbH, 70442 Stuttgart (DE)
(72) Erfinder: LEDERMANN, Bernhard, 70499 Stuttgart (DE)
(86) Internationale Anmeldenummer: PCT/EP2013/070319
(87) Internationale Veröffentlichungsnummer: WO 2014/063903

(56) Entgegenhaltungen:
- EP-A2- 0 833 148
- EP-A2- 0 964 246
- WO-A1-2004/053475
- DE-A1-102009 045 445

## Beschreibung

### Stand der Technik

Die Erfindung betrifft ein Verfahren zur Diagnose eines Referenzkanals einer Breitband-Lambdasonde, welche zur Bestimmung eines Sauerstoffgehalts in einem Abgas verwendet wird, wobei mindestens ein Sensorelement mit einer Pumpzelle und einer Nernstzelle verwendet wird, wobei in einem Messmodus zur Bestimmung des Sauerstoffgehalts im Abgas ein geregelter Pumpstrom durch die Pumpzelle fließt und damit ein Austausch von Sauerstoffionen zwischen einem Messraum und dem Abgas erreicht und damit ein Lambda-Wert in dem Messraum auf einen Wert von 1 geregelt wird, wobei der Lambda-Wert in dem Messraum durch die Nernstzelle überwacht wird, und wobei der Wert des dazu notwendigen Pumpstroms abhängig von der Sauerstoffkonzentration und somit dem zu bestimmenden Lambda-Wert des Abgases ist, wobei das Sensorelement mit seinen Elektroden mit einer Steuereinheit zur Ansteuerung und Auswertung der Breitband-Lambdasonde verbunden ist, wobei neben dem Messmodus, auch mindestens ein Diagnosemodus vorgesehen ist.

Die Erfindung betrifft weiterhin eine entsprechende Vorrichtung zur Durchführung des erfindungsgemäßen Verfahrens.

Lambdasonden werden zum Beispiel im Abgasstrang von Brennkraftmaschinen zur Messung des Sauerstoffgehalts des Abgases eingesetzt, um die Aufbereitung des Kraftstoff-Luft-Gemisches der Brennkraftmaschine zu steuern. Sie nutzen die Eigenschaft eines aus einer Zirkondioxid-Keramik aufgebauten Festkörperelektrolyten, der ab einer Temperatur von ca. 300°C für Sauerstoffionen durchlässig ist. An zumindest zwei Seiten des Festkörperelektrolyten sind Platinelektroden aufgebracht. Ist eine der Seiten einem Abgas mit vermindertem Sauerstoff-Partialdruck ausgesetzt und die andere Seite einem Referenzgas, beispielsweise Luft, so diffundieren Sauerstoffionen durch den Festkörperelektrolyten. Die dadurch hervorgerufene Spannungsdifferenz zwischen den beiden Elektroden einer solchen Konzentrationszelle ist durch die Nernst-Gleichung beschrieben und kann zur Bestimmung des Lambda-Wertes des Abgases in einem engen Fenster um Lambda = 1 verwendet werden. Der Lambda-Wert stellt das vorliegende Luft-/Kraftstoff-Verhältnis zu einem stöchiometrischen Luft-/Kraftstoff-Verhältnis dar.

Durch Anlegen einer externen Spannung an die Elektroden können Sauerstoffionen durch den Festkörperelektrolyten gepumpt werden. Diese Eigenschaft wird bei sogenannten Breitband-Lambdasonden genutzt, um den Lambdawert in einem großen Bereich von magerem zu fettem Abgas bestimmen zu können. Dazu ist eine Elektrode, die äußere Pumpelektrode, dem Abgas zugewandt. Die zweite Elektrode ist als innere Pumpelektrode über eine Diffusionsbarriere mit dem Abgas verbunden. Die zweite Elektrode kann in einem Messraum in dem Festkörperelektrolyten, welcher durch einen Diffusionskanal und die Diffusionsbarriere mit dem Abgas verbunden ist, angeordnet sein. In einem alternativen Aufbau sind beide Elektroden auf der dem Abgas zugewandten Seite des Festkörperelektrolyten angeordnet, wobei die innere Pumpelektrode durch eine aufgebrachte Diffusionsbarriere-Schicht abgedeckt ist.

Äußere Pumpelektrode, innere Pumpelektrode und der dazwischen liegende Festkörperelektrolyt bilden eine sogenannte Pumpzelle. Durch Anlegen einer Spannung werden Sauerstoff-Ionen von der inneren Pumpelektrode zu der äußeren Pumpelektrode transportiert. Bei ausreichend hoher Spannung stellt sich dabei ein Grenzstrom ein, der von der Sauerstoffdiffusion durch die Diffusionsbarriere bestimmt ist. Die Sauerstoffdiffusion und damit der zur Bestimmung des Lambda-Wertes gemessene Grenzstrom sind direkt von dem Sauerstoff-Partialdruck in dem Abgas sowie von den Diffusionseigenschaften der Diffusionsbarriere abhängig. Bei bekannten Diffusionseigenschaften der Diffusionsbarriere kann das Lambda des Abgases aus dem Grenzstrom bestimmt werden.

Ein weiterer, als zweizellige Breitband-Lambdasonde bekannter Aufbau ist aus einer Kombination einer Pumpzelle und einer Konzentrationszelle gebildet. Dabei ist die äußere Pumpelektrode dem Abgas zugewandt und die innere Pumpelektrode in einem durch die Diffusionsbarriere mit dem Abgas verbundenen Messraum angeordnet. In dem Messraum befindet sich weiterhin eine als Messelektrode bezeichnete erste Elektrode der Konzentrationszelle. In einem gesonderten Referenzkanal ist als zweite Elektrode der Konzentrationszelle eine Referenzelektrode auf dem Festkörperelektrolyten aufgebracht. Der Referenzkanal ist mit einem Referenzgas mit definiertem Sauerstoffgehalt, vorzugsweise durch eine äußere Öffnung, mit Luft gefüllt.

Bei einer solchen zweizelligen Breitband-Lambdasonde wird durch die Pumpzelle der Lambda-Wert in dem Messraum durch Ein- oder Auspumpen von Sauerstoffionen auf ein Lambda von vorzugsweise 1 eingestellt. Der Lambda-Wert in dem Messraum wird dazu über die Konzentrationszelle gemessen und durch eine entsprechende Regelung des Pumpstroms durch die Pumpzelle auf λ=1 geregelt. Der dazu notwendige Pumpstrom ist abhängig von der durch die Diffusionsbarriere in den Messraum diffundierenden Sauerstoffmenge und somit von dem Lambda des Abgases und den Diffusionseigenschaften der Diffusionsbarriere. Bei bekannten Eigenschaften der Diffusionsbarriere kann aus dem Pumpstrom das Lambda des Abgases bestimmt werden.

Die Diffusionseigenschaften der Diffusionsbarrieren unterliegen einer starken Fertigungsstreuung. Die individuellen Diffusionseigenschaften müssen daher bei der Auswertung der Signale der Breitband-Lambdasonde berücksichtigt werden.

Ein Beispiel einer Breitband-Lambdasonde ist in der DE 10 2008 002 734 A1 beschrieben.

Vorrichtungen zum Betreiben einer Breitband-Lambdasonde, welche als Auswerte- und Steuereinheiten dienen, sind beispielsweise in der DE 10 2010 000 663 A1 beschrieben. Der Grundgedanke, der Aufbau und die Grundfunktion der in dieser Schrift beschrieben Steuereinheit entspricht im Wesentlichen der in der Schrift DE 10 2008 001 697 A1 der Anmelderin dargestellten Auswerte- und Steuereinheit für eine Breitband-Lambdasonde. Dabei ist die Steuereinheit in Form eines ASIC ausgeführt und unter dem Namen CJ 125 bzw. CJ 135 bekannt (s. Produkt Information der Anmelderin mit dem Titel "CJ135 - Lambda Probe Interface IC").

In der DE 10 2010 039 188 A1 wird ein Verfahren zur Erfassung mindestens einer Eigenschaft eines Gases in einem Messraum beschrieben, insbesondere zum Nachweis einer Gaskomponente des Gases. Bei dem Verfahren wird mindestens ein Sensorelement mit mindestens einer Zelle verwendet. Die Zelle weist mindestens eine erste Elektrode, mindestens eine zweite Elektrode und mindestens einen die erste Elektrode und die zweite Elektrode verbindenden Festelektrolyten auf. Die erste Elektrode ist mit Gas aus dem Messraum beaufschlagbar. Die zweite Elektrode steht mit mindestens einem Referenzgasraum in Verbindung, welcher eingerichtet ist, um einen Vorrat einer Gaskomponente des Gases zu speichern. Das Verfahren umfasst mindestens zwei Betriebsmodi:
- Mindestens einen Messmodus, wobei in dem Messmodus die Zelle als Pumpzelle betrieben wird und aus mindestens einem Pumpstrom durch die Pumpzelle auf die Eigenschaft geschlossen wird, und
- Mindestens einen Diagnosemodus, wobei in dem Diagnosemodus eine Speicherfähigkeit des Referenzgasraums überprüft wird, wobei mindestens eine durch eine an der Zelle anliegende Nernst-Spannung beeinflusste Messgröße erfasst wird und aus der Messgröße auf die Speicherfähigkeit geschlossen wird.

Ein Großteil der aktuell auf dem Markt erhältlichen Lambdasonden benötigt für Ihr Funktionsprinzip eine sogenannte Luft-Referenz (eigentlich Sauerstoff-Referenz), die sich dadurch auszeichnet, dass der Sauerstoffgehalt dieser Luft-Referenz bei 21% Sauerstoff oder höher liegt. Alle Verfahren, die diese Luft-Referenz zur Verfügung stellen, beinhalten gewisse Risiken, dass diese nicht ausreichend Sauerstoff beinhalten bzw. durch Elemente mit negativer Sauerstoffwertigkeit "vergiftet" sind. Als Konsequenz dieser fehlerhaften Referenz kommt es zu einer Verschiebung der Lambda-Kennlinie und in logischer Konsequenz zu einer fehlerhaften Lambda-Bestimmung und bisweilen zu unerwünschten Fehlereinträgen in der Software des Motorsteuergerätes. Eine Diagnose der Luft-Referenz ist in konventionellen Auswerteschaltungen meist nicht möglich und es muss das oben dargestellte Restrisiko in Kauf genommen werden.

Es ist daher Aufgabe der Erfindung, ein entsprechendes Verfahren bereitzustellen, mit dem eine einfache und schnelle Diagnose der oben beschriebenen Luft-Referenz von Breitband-Lambdasonden durchgeführt werden kann.

Weiterhin ist es Aufgabe der Erfindung, eine entsprechende Vorrichtung zur Durchführung des Verfahrens bereit zu stellen.

### Offenbarung der Erfindung

Die das Verfahren betreffende Aufgabe wird dadurch gelöst, dass während des Diagnosemodus eine Umschaltung am Pumpstrom-Regelkreis vorgenommen wird und über eine Spannungsmessung eine Diagnose der Güte einer Luft-Referenz im Referenzkanal durchgeführt wird. Mit dem Verfahren kann ein fehlerhafter Referenzkanal im Sensorelement diagnostiziert und damit ein möglicherweise falscher Lambda-Wert vermieden werden, was die Betriebssicherheit einer Lambdaregelung bei einer Brennkraftmaschine erhöht und Schadstoffemissionen vermeiden hilft.

Dabei ist vorgesehen, dass der Pumpstrom-Regelkreis derart modifiziert wird, dass die Sauerstoffkonzentrationen im Sensorelement derart verändert werden, dass eine Vergleichsmessung zwischen zwei Stellen mit hoher Sauerstoffkonzentration möglich ist. Erhält man ein Messergebnis, dass als fehlerhafte Referenz zu deuten ist, können sich als Konsequenz mehrere Szenarien ergeben. Zum einen kann durch einen dauerhaft hohen Referenzpumpstrom versucht werden, die Luft-Referenz zu reinigen und mit Sauerstoff zu befüllen. Andererseits kann durch einen Fehlereintrag angezeigt werden, dass ein Sondentausch zwingend erforderlich ist.

In bevorzugter Verfahrensvariante ist vorgesehen, dass für die Diagnose ein maximal möglicher negativer Pumpstrom eingestellt wird. Dies ermöglicht eine Befüllung des Messraums mit einem Überdruck an Sauerstoffionen. Dieser hohe Sauerstoffpartialdruck führt bereits nach kurzer Zeit (typischerweise bereits nach < 250 ms) zu einem statischen Zustand, dessen Ausgleich aller neu hinzugeführten Sauerstoffionen über Diffusion und Strömung durch eine Diffusionsbarriere in das Abgas erfolgt. Nach Erreichen des stationären Zustandes kann die eingangs erwähnte Spannungsmessung durchgeführt werden, ohne dass zeitliche Änderungen diese stören würden.

Zudem kann dies auch durch eine Anforderung an eine negative Regelspannung an die Nernstzelle erreicht werden.

Dabei ist vorgesehen, dass während der Diagnosephase, in der die sich einstellende Nernst-Spannung einen stationären Wert angenommen hat, diese ausgewertet wird und bei einer Nernst-Spannung von + 50 mV bis +150 mV von einem intakten Referenzkanal mit intakter Luft-Referenz und bei einer sich einstellenden demgegenüber niedrigeren oder negativen Spannung von einer fehlerhaften Luft-Referenz ausgegangen wird. Dies kann einfach mittels entsprechender Komparatoren überwacht werden.

Bei einem erkannten Fehler kann in einer Verfahrensvariante eine Beschaltung des Sensorelements zum intensiven Befüllen des Referenzkanals mit Luft bzw. Sauerstoff angefordert werden, während der gemessene Lambda-Wert in unmittelbarer zeitlicher Nähe zur Diagnosephase als ungültig gekennzeichnet werden kann. Damit kann das Sensorelement zumindest teilweise wieder regeneriert bzw. "entgiftet" werden.

Wird die gemessene Nernst-Spannung insbesondere im Bereich von 0 bis 500 mV durch weitere Maßnahmen plausibilisiert, ergibt sich damit eine besonders hohe Diagnosesicherheit.

Aufgrund der recht kurzen Diagnosezeitdauer von maximal einer Sekunde, typischerweise liegt diese bei ca. 0,5 Sekunden, wird die Lambda-Messung nur kurzzeitig unterbrochen, so dass weiterhin eine nahezu lückenlose Abgasüberwachung ermöglicht wird.

Bevorzugt wird das Diagnoseverfahren mit seinen zuvor beschriebenen Verfahrensvarianten zu Beginn der Lebensdauer einer Breitband-Lambdasonde und/ oder, in bestimmten Abständen, während der Lebenszeit durchgeführt. Am Anfang der Lebensdauer lässt sich damit beispielsweise erkennen, ob die Abgassonde zu lange bzw. unsachgemäß gelagert wurde. Eine Anwendung während der Lebenszeit bietet den Vorteil, dass eine Alterung oder eine mechanische Beschädigung erkannt werden kann. Temporär "vergiftete" Sonden können damit regeneriert werden, bevor deren Lambdasignal verwendet wird. Dies kann inkorrekte schwer zuzuordnende Folgefehler bei Funktionalitäten, die das Lambda-Signal nutzen, verhindern.

Vorteilhaft ist es weiterhin, dass die Diagnose lediglich einmal während eines Fahrzyklus, insbesondere am Anfang oder am Ende eines Fahrzyklus durchgeführt werden muss.

Die die Vorrichtung betreffende Aufgabe wird dadurch gelöst, dass die Steuereinheit Einrichtungen zur Durchführung des Verfahrens mit seinen zuvor beschriebenen Verfahrensvarianten, insbesondere Umschalteinrichtungen zur Änderung einer Pumpstrom-Regelung des Pumpstromes durch die Pumpzelle sowie Komparatoren zur Auswertung einer Nernst-Spannung an der Nernstzelle aufweisen. Die Steuereinheit kann dabei zumindest teilweise integraler Bestandteil einer übergeordneten Motorsteuerung einer Brennkraftmaschine sein. Die Funktionalität kann dabei zumindest teilweise oder komplett software-basiert ausgeführt sein, was insbesondere eine Adaption recht einfach ermöglicht.

Weist, wie dies eine besonders bevorzugte Vorrichtungsvariante vorsieht, die Steuereinheit ein CJ 135 ASIC-Baustein auf, ist der applikative Aufwand zur Anwendung des Diagnoseverfahrens vergleichsweise gering, da dieser Baustein bereits entsprechende umfangreiche Einstellmöglichkeiten aufweist, um die nötigen Umschaltungen vorzunehmen, die gegenüber dem Normalbetrieb vorgenommen werden müssen.

Die Erfindung wird im Folgenden anhand eines in den Figuren dargestellten Ausführungsbeispiels näher erläutert. Es zeigen:
Figur 1 in schematischer Darstellung ein Sensorelement einer zweizelligen Breitband-Lambdasonde mit einem Steckergehäuse und
Figur 2 ein Signal-Verlaufsdiagramm für eine Nernst-Spannung und einen Pumpstrom.

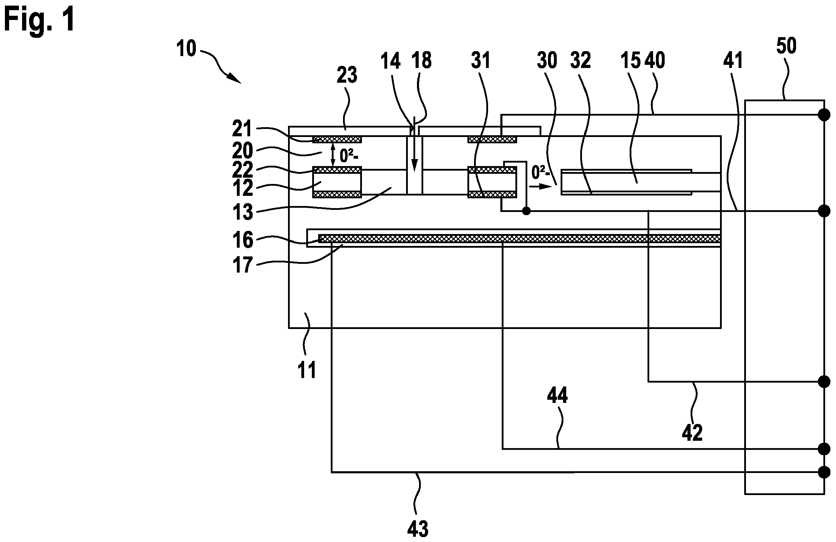

Figur 1 zeigt in schematischer Darstellung ein Sensorelement 10 einer planaren, zweizelligen Breitband-Lambdasonde zur Bestimmung eines Lambdawertes eines Abgases 18 mit einem Steckergehäuse 50. Das Sensorelement 10 ist im Wesentlichen aus einem Festkörperelektrolyten 11 aufgebaut. Es enthält eine Pumpzelle 20, eine Nernstzelle 30 und ein Heizelement 16. Der Festkörperelektrolyt 11 ist in der Darstellung als einheitlicher Körper aus Sauerstoffionen leitendem Zirkondioxid gezeigt, er kann jedoch im Aufbau aus einer Mehrzahl von Festelektrolytschichten aufgebaut sein.

Die Pumpzelle 20 besteht aus einer äußeren Pumpelektrode 21 und einer in einem Messraum 12 angeordneten inneren Pumpelektrode 22. Die äußere Pumpelektrode 21 ist, abgedeckt durch eine Schutzschicht 23, dem Abgas 18 einer Brennkraftmaschine ausgesetzt. Die äußere Pumpelektrode 21 und die innere Pumpelektrode 22 sind ringförmig um einen Diffusionskanal 14 angeordnet. Der Diffusionskanal 14 führt das Abgas über eine Diffusionsbarriere 13 dem Messraum 12 zu.

Auf der der inneren Pumpelektrode 22 gegenüberliegenden Seite des Messraums 12 ist eine Messelektrode 31 angeordnet. Die Messelektrode 31 bildet zusammen mit einer in einem Referenzkanal 15 angeordneten Referenzelektrode 32 und dem dazwischen liegenden Festkörperelektrolyten 11 die Nernstzelle 30 oder Konzentrationszelle. Der Referenzkanal 15 ist mit einem luftdurchlässigen Material gefüllt und zur Außenluft als Referenzgas geöffnet. Im Falle einer gepumpten Referenz kann der Referenzkanal 15 auch vollständig oder weitgehend geschlossen und beispielsweise mit Zirkonoxid gefüllt sein. Der Referenzkanal 15 stellt eine Luft-Referenz oder auch Sauerstoff-Referenz dar.

Das Heizelement 16 ist durch ein Isolationsmaterial 17 von dem Festkörperelektrolyten 11 elektrisch getrennt.

Die äußere Pumpelektrode 21 ist über einen Anschluss APE 40 mit dem Steckergehäuse 50 verbunden. Die innere Pumpelektrode 22 und die Messelektrode 31 sind parallel geschaltet und über einen gemeinsamen Anschluss IPN 41 zum Steckergehäuse 50 geführt. Die Referenzelektrode 32 ist über einen Anschluss RE 42 und das Heizelement 16 über einen ersten Heizanschluss 43 und einen zweiten Heizschluss 44 an das Steckergehäuse 50 angeschlossen.

Die Elektroden, d.h. die innere und äußere Pumpelektrode 21, 22, die Referenzelektrode 32 und die Messelektrode 31 sind aus Platin gefertigt.

Im Betrieb der Breitband-Lambdasonde diffundiert Abgas über den Diffusionskanal 14 und die Diffusionsbarriere 13 in den Messraum 12. Über die Nernstzelle 30 wird der Lambdawert in dem Messraum 12 durch Messung der Nernstspannung zwischen der Messelektrode 31 und der Referenzelektrode 32 bestimmt. Die Nernstzelle 30 ermöglicht dabei die Bestimmung des Lambdas in einem engen Messfenster um Lambda = 1. Durch Anlegen einer entsprechen polarisierten Spannung zwischen der äußeren und der inneren Pumpelektrode 21, 22 können Sauerstoffionen durch den Festkörperelektrolyten 11 von dem Abgas in dem Messraum 12 oder aus dem Messraum 12 zu dem Abgas gepumpt werden.

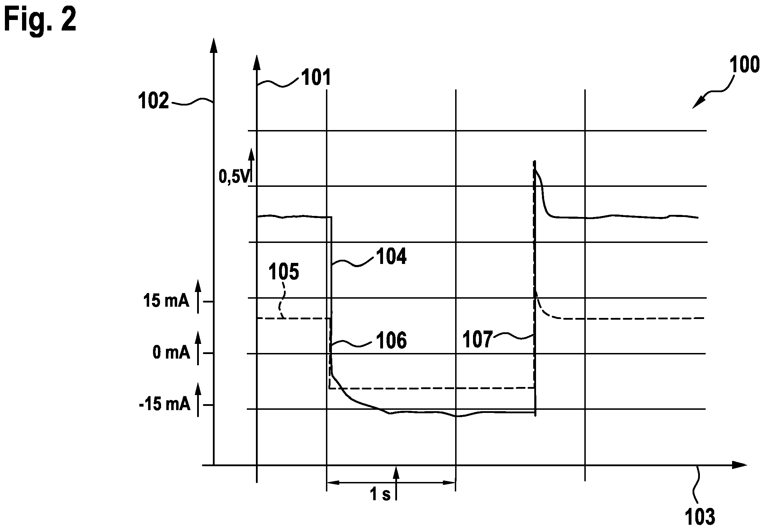

Die Nernstzelle 30 wird während des Betriebs an einer Steuereinheit, welche z.B. ein CJ 135 ASIC sein kann, wie diese in der DE 10 2008 001 697 A1 beschrieben ist, unter Ausnutzung eines Referenzpumpstroms auf eine Spannung von 450 mV geregelt, so dass der Messraum 12 (Lambda-1-Hohlraum) innerhalb der Lambdasonde im Wesentlichen als sauerstofffrei anzusehen ist. Dies geschieht durch Anlegen einer Stromquelle an die Pumpzelle 20. Durch eine geeignete Regelung des zwischen den Pumpelektroden 21, 22 fließenden Pumpstroms 102 (siehe Figur 2) und damit des Austauschs von Sauerstoffionen zwischen dem Messraum 12 und dem Abgas wird der Lambda-Wert in dem Messraum 12 auf einen Wert von 1 geregelt. Der Lambda-Wert in dem Messraum 12 wird dabei durch die Nernstzelle 30 überwacht. Der Wert des dazu notwendigen Pumpstroms ist abhängig von der Sauerstoffkonzentration und somit dem zu bestimmenden Lambda-Wert des Abgases sowie von den Diffusionseigenschaften der Diffusionsbarriere 13. Essentielle Bedingung für das Funktionieren des Konzeptes ist, dass durch den oben erwähnten Referenzpumpstrom eine Kammer mit sehr hohem Sauerstoffgehalt, der Luft-Referenz, zur Verfügung gestellt wird.

Ist diese Luft-Referenz bzw. Sauerstoff-Referenz nicht vorhanden, ist der angelegte Pumpstrom 102 (siehe Figur 2) im Sinne der Sondendefinition nicht mehr als gültiges Maß für den Sauerstoffgehalt des Abgases 18 anzusehen.

Während der Diagnose der Luft-Referenz kann die Steuereinheit (z. B. CJ 135 ASIC) im Diagnose-Zeitraum dazu gebracht werden, einen maximal negativen Pumpstrom 102 (siehe Figur 2) einzustellen, der den Messraum 12 (Lambda-1-Hohlraum) mit einem Überdruck an Sauerstoffionen füllt. Dieser kann z.B. in dieser Phase in der Größenordnung von -10 mA bis -15 mA betragen. Dies kann entweder über eine Anforderung einer negativen Regelspannung an der Nernstzelle 30 oder durch das Anfordern des gestellten Pumpstrom-Betriebes in der Steuereinheit geschehen.

Der oben beschriebene Sauerstoff-Überdruck (hoher Sauerstoff-Partialdruck) führt nach kurzer Zeit zu einem statischen Zustand, dessen Ausgleich aller neu hinzu geführten Sauerstoffionen über Diffusion und Strömung durch die Diffusionsbarriere 13 in das Abgas 18 erfolgt.

In Figur 2 sind in einem Signal-Verlaufsdiagramm 100 ein Nernst-Spannungsverlauf 104 und ein Pumpstromverlauf 105 dargestellt, wobei die Abszisse die Zeit 103 und die Ordinate die Höhe der Nernst-Spannung 101 und des Pumpstroms 102 beschreiben.

Ausgehend von einer konstant geregelten Nernst-Spannung 101 von 450 mV mit einem entsprechenden Pumpstrom 102 stellt sich nach der o.g. Maßnahme bei einer ersten Flanke 106 innerhalb von etwa 250 ms ein neuer statischer Zustand ein. Dabei bildet sich zwischen dem Messraum 12 (Lambda-1-Hohlraum) und der Luft-Referenz eine Nernst-Spannung 101 aus, die im Bereich von ca. +100 mV liegt, da typischerweise der Sauerstoffpartialdruck in der Luft-Referenz noch höher liegt als im nun mit Sauerstoff gefüllten Messraum 12. Beim Normalbetrieb liegt diese bei +450 mV.

Bildet sich eine deutlich negative Spannung aus, so kann von einer fehlerhaften Luft-Referenz ausgegangen werden und, falls diese eigentlich funktional sein sollte, ein Sondenfehler erkannt werden oder eine Reaktion zum intensiven Befüllen der Luft-Referenz angefordert werden, während der das Lambda-Signal als ungültig anzusehen ist.

Das Zurückschalten in den Normalbetrieb, was eine zweite Flanke 107 im Nernst-Spannungsverlauf 104 bzw. im Pumpstromverlauf 105 anzeigt, dauert nur etwa 200 ms, bis der Pumpstrom 102 wieder den korrekten Wert anzeigt und die Nernst-Spannung 101 wieder auf einen Wert von 450 mV eingeregelt ist. Die Dauer eines Diagnose-Zyklus liegt insgesamt im Bereich von etwa einer Sekunde und kleiner (typischerweise < 500 ms). Die Diagnose muss nicht öfter als 1 mal pro Fahrzyklus durchgeführt werden. Ein möglicher Zeitpunkt für die Diagnose ist z.B. zu Beginn oder zum Ende eines Fahrbetriebs des Fahrzeugs.

## Patentansprüche

1. Verfahren zur Diagnose eines Referenzkanals (15) einer Breitband-Lambdasonde, welche zur Bestimmung eines Sauerstoffgehalts in einem Abgas (18) verwendet wird, wobei mindestens ein Sensorelement (10) mit einer Pumpzelle (20) und einer Nernstzelle (30) verwendet wird, wobei in einem Messmodus zur Bestimmung des Sauerstoffgehalts im Abgas (18) ein geregelter Pumpstrom (102) durch die Pumpzelle (20) fließt und damit ein Austausch von Sauerstoffionen zwischen einem Messraum (12) und dem Abgas (18) erreicht und damit ein Lambda-Wert in dem Messraum (12) auf einen Wert von 1 geregelt wird, wobei der Lambda-Wert in dem Messraum (12) durch die Nernstzelle (30) überwacht wird, und wobei der Wert des dazu notwendigen Pumpstroms (102) abhängig von der Sauerstoffkonzentration und somit dem zu bestimmenden Lambda-Wert des Abgases (18) ist, wobei das Sensorelement (10) mit seinen Elektroden mit einer Steuereinheit zur Ansteuerung und Auswertung der Breitband-Lambdasonde verbunden ist, wobei neben dem Messmodus, auch mindestens ein Diagnosemodus vorgesehen ist, **dadurch gekennzeichnet, dass** während des Diagnosemodus eine Umschaltung am Pumpstrom-Regelkreis vorgenommen wird und über eine Spannungsmessung eine Diagnose der Güte einer Luft-Referenz im Referenzkanal (15) durchgeführt wird und dass für die Diagnose ein stark negativer Pumpstrom (102) eingestellt wird und dass die sich in der Diagnosephase einstellende Nernstspannung ausgewertet wird.

2. Verfahren nach Anspruch 1, **dadurch gekennzeichnet, dass** der Pumpstrom-Regelkreis derart modifiziert wird, dass die Sauerstoffkonzentrationen im Sensorelement (10) derart verändert werden, dass eine Vergleichsmessung zwischen zwei Stellen mit hoher Sauerstoffkonzentration möglich ist.

3. Verfahren nach Anspruch 1 oder 2, **dadurch gekennzeichnet, dass** eine Anforderung an eine negative Regelspannung an die Nernstzelle (30) gestellt wird.

4. Verfahren nach einem der Ansprüche 1 bis 3, dass während der Diagnosephase, in der die sich einstellende Nernst-Spannung (101) einen annähernd stationären Wert angenommen hat, diese ausgewertet wird und bei einer Nernst-Spannung (101) von + 50 mV bis +150 mV von einem intakten Referenzkanal (15) mit intakter Luft-Referenz und bei einer sich einstellenden demgegenüber niedrigeren oder negativen Spannung von einer fehlerhaften Luft-Referenz ausgegangen wird.

5. Verfahren nach Anspruch 4, **dadurch gekennzeichnet, dass** bei einem erkannten Fehler eine Beschaltung des Sensor-Elements (10) zum intensiven Befüllen des Referenzkanals (15) mit Luft bzw. Sauerstoff angefordert wird, während der gemessene Lambda-Wert in unmittelbarer zeitlicher Nähe zur Diagnosephase als ungültig gekennzeichnet wird.

6. Verfahren nach einem der Ansprüche 1 bis 5, **dadurch gekennzeichnet, dass** die gemessene Nernst-Spannung (101) insbesondere im Bereich von 0 bis 500 mV durch weitere Maßnahmen plausibilisiert wird.

7. Verfahren nach einem der Ansprüche 1 bis 6, **dadurch gekennzeichnet, dass** diese Diagnose innerhalb einer Diagnosezeitdauer von einer Sekunde durchgeführt wird.

8. Verfahren nach einem der Ansprüche 1 bis 7, **dadurch gekennzeichnet, dass** das Diagnoseverfahren zu Beginn der Lebensdauer einer Breitband-Lambdasonde und/ oder, in bestimmten Abständen, während der Lebenszeit durchgeführt wird.

9. Verfahren nach einem der Ansprüche 1 bis 8, **dadurch gekennzeichnet, dass** die Diagnose einmal während eines Fahrzyklus, insbesondere am Anfang oder am Ende eines Fahrzyklus durchgeführt wird.

10. Vorrichtung zur Diagnose eines Referenzkanals (15) einer Breitband-Lambdasonde zur Bestimmung des Sauerstoffgehalts in einem Abgas (18), wobei mindestens ein Sensorelement (10) mit einer Pumpzelle (20) und einer Nernstzelle (30) vorgesehen ist und das Sensorelement (10) mit seinen Elektroden mit einer Steuereinheit zur Ansteuerung und Auswertung der Breitband-Lambdasonde verbunden ist, **dadurch gekennzeichnet, dass** die Steuereinheit Einrichtungen zur Durchführung des Verfahrens nach einem der Verfahrensansprüche 1 bis 9, insbesondere Umschalteinrichtungen zur Änderung einer Pumpstrom-Regelung eines Pumpstromes (102) durch die Pumpzelle (20) sowie Komparatoren zur Auswertung einer Nernst-Spannung (101) an der Nernstzelle (30) aufweisen.

11. Vorrichtung nach Anspruch 10, **dadurch gekennzeichnet, dass** die Steuereinheit einen CJ 135 ASIC-Baustein aufweist.

## Claims

1. Method for diagnosing a reference channel (15) of a broadband lambda probe, which is used to determine the oxygen content in an exhaust gas (18), wherein at least one sensor element (10) having a pump cell (20) and a Nernst cell (30) is used, wherein, in a measuring mode, to determine the oxygen content in the exhaust gas (18), a regulated pump current (102) flows through the pump cell (20) and thus achieves an exchange of oxygen ions between a measuring space (12) and the exhaust gas (18) and thus regulates a lambda value in the measuring space (12) to a value of 1, wherein the lambda value in the measuring space (12) is monitored by the Nernst cell (30), and wherein the value of the pump current (102) that is necessary therefor is dependent on the oxygen concentration and hence on the lambda value of the exhaust gas (18) to be determined, wherein the sensor element (10) is connected by way of the electrodes thereof to a control unit for actuating and evaluating the broadband lambda probe, wherein, in addition to the measuring mode, at least one diagnosis mode is also provided, **characterized in that**, during the diagnosis mode, a changeover at the pump-current control loop is performed and a diagnosis is made about the quality of an air reference in the reference channel (15) by means of a voltage measurement and **in that**, for the diagnosis, a significantly negative pump current (102) is set and **in that** the Nernst voltage ensuing in the diagnosis phase is evaluated.

2. Method according to Claim 1, **characterized in that** the pump-current control loop is modified in such a way that the oxygen concentrations in the sensor element (10) are changed in such a way that a comparison measurement between two locations with a high oxygen concentration is possible.

3. Method according to Claim 1 or 2, **characterized in that** a negative control voltage is requested from the Nernst cell (30).

4. Method according to one of Claims 1 to 3, that, during the diagnosis phase, in which the ensuing Nernst voltage (101) has assumed an approximately stationary value, said Nernst voltage is evaluated and, in the event of a Nernst voltage (101) of + 50 mV to + 150 mV, an intact reference channel (15) with an intact air reference is assumed and, in the event of an ensuing voltage that is lower or negative in comparison, a faulty air reference is assumed.

5. Method according to Claim 4, **characterized in that**, in the event of an identified fault, connection of the sensor element (10) is requested to intensively fill the reference channel (15) with air or oxygen, whereas the measured lambda value is denoted as invalid in immediate temporal proximity to the diagnosis phase.

6. Method according to one of Claims 1 to 5, **characterized in that** the plausibility of the measured Nernst voltage (101) is checked, in particular in the range of from 0 to 500 mV, by further measures.

7. Method according to one of Claims 1 to 6, **characterized in that** said diagnosis is carried out within a diagnosis period of one second.

8. Method according to one of Claims 1 to 7, **characterized in that** the diagnosis method is carried out at the beginning of the lifetime of a broadband lambda probe and/or at specific intervals during the lifetime.

9. Method according to one of Claims 1 to 8, **characterized in that** the diagnosis is carried out once during a drive cycle, in particular at the start or at the end of a drive cycle.

10. Apparatus for diagnosing a reference channel (15) of ;a broadband lambda probe to determine the oxygen content in an exhaust gas (18), wherein at least one sensor element (10) having a pump cell (20) and a Nernst cell (30) is provided and the sensor element (10) is connected by way of the electrodes thereof to a control unit for actuating and evaluating the broadband lambda probe, **characterized in that** the control unit has devices for carrying out the method according to one of Method Claims 1 to 9, in particular changeover devices for changing a pump-current control process of a pump current (102) through the pump cell (20) and comparators for evaluating a Nernst voltage (101) at the Nernst cell (30).

11. Apparatus according to Claim 10, **characterized in that** the control unit has a CJ 135 ASIC component.

## Revendications

1. Dispositif de diagnostic d'un canal de référence (15) d'une sonde lambda à large bande utilisée pour déterminer une teneur en oxygène d'un gaz d'échappement (18), dans lequel au moins un élément détecteur (10) comportant une cellule de pompage (20) et une cellule de Nernst (30) est utilisé, dans lequel, dans un mode de mesure servant à déterminer la teneur en oxygène du gaz d'échappement (18), un courant de pompage régulé (102) passe à travers la cellule de pompage (20), ce qui provoque un échange d'ions oxygène entre une chambre de mesure (12) et le gaz d'échappement (18) et permet de réguler une valeur lambda égale à 1 dans la chambre de mesure (12), dans lequel la valeur lambda dans la chambre de mesure (12) est surveillée par la cellule de Nernst (30), et dans lequel la valeur du courant de pompage (102) nécessaire pour cela dépend de la concentration en oxygène et par conséquent, de la valeur lambda à déterminer du gaz d'échappement (18), dans lequel l'élément détecteur (10) est relié par ses électrodes à une unité de commande destinée à attaquer et à analyser la sonde lambda à large bande, dans lequel, outre le mode de mesure, il est également prévu un mode de diagnostic, **caractérisé en ce qu'**un basculement sur le circuit de régulation de courant de pompage est effectué pendant le mode de diagnostic et un diagnostic de la qualité d'une référence air dans le canal de référence (15) est réalisé au moyen d'une mesure de tension et **en ce qu'**un courant de pompage (102) fortement négatif est réglé pour le diagnostic et **en ce que** la tension de Nernst s'établissant lors de la phase de diagnostic est analysée.

2. Procédé selon la revendication 1, **caractérisé en ce que** le circuit de régulation de courant de pompage est modifié de manière à ce que les concentrations en oxygène dans l'élément détecteur (10) soient modifiées pour rendre possible une mesure de comparaison entre deux points présentant une concentration en oxygène plus élevée.

3. Procédé selon la revendication 1 ou 2, **caractérisé en ce qu'**une demande de tension de régulation négative est réglée sur la cellule de Nernst (30) .

4. Procédé selon l'une des revendications 1 à 3, que, pendant la phase de diagnostic au cours de laquelle la tension de Nernst (101) qui s'établit a atteint une valeur pratiquement stationnaire, cette dernière est analysée et lorsque la tension de Nernst (101) est comprise entre +50 mV et +150 mV, il est considéré que le canal de référence (15) est intact avec une référence air intacte et lorsqu'une tension inférieure à cela ou négative s'établit, il est considéré que la référence air est défectueuse.

5. Procédé selon la revendication 4, **caractérisé en ce que**, dans le cas d'un défaut détecté, un circuit de l'élément détecteur (10) est nécessaire pour un remplissage intensif du canal de référence (15) avec de l'air ou de l'oxygène, tandis que la valeur lambda mesurée est **caractérisée** comme étant invalide à proximité immédiate dans le temps de la phase de diagnostic.

6. Procédé selon l'une des revendications 1 à 5, **caractérisé en ce que** la tension de Nernst (101) mesurée est rendue plausible, en particulier dans la plage de 0 à 500 mV, par d'autres actions.

7. i Procédé selon l'une des revendications 1 à 6, **caractérisé en ce que** ledit diagnostic est réalisé au cours d'un intervalle de temps de diagnostic d'une seconde.

8. Procédé selon l'une des revendications 1 à 7, **caractérisé en ce que** le procédé de diagnostic est réalisé au début de la durée de vie d'une sonde lambda à large bande et/ou à des intervalles déterminés, pendant le temps de vie.

9. Procédé selon l'une des revendications 1 à 8, **caractérisé en ce que** le diagnostic est réalisé une fois pendant un cycle de conduite, en particulier au début ou à la fin d'un cycle de conduite.

10. Dispositif de diagnostic d'un canal de référence (15) d'une sonde lambda à large bande utilisée pour déterminer une teneur en oxygène d'un gaz d'échappement (18), dans lequel il est prévu au moins un élément détecteur (10) comportant une cellule de pompage (20) et une cellule de Nernst (30) et l'élément détecteur (10) est relié par ses électrodes à une unité de commande destinée à attaquer et à analyser la sonde lambda à largeur bande, caractérisé en ce l'unité de commande comporte des dispositifs destinés à mettre en oeuvre le procédé selon l'une des revendications de procédé 1 à 9, en particulier des dispositifs de basculement destinés à modifier une régulation de courant de pompage d'un courant de pompage (102) passant à travers la cellule de pompage (20) ainsi que des comparateurs destinés à analyser une tension de Nernst (101) au niveau de la cellule de Nernst (30).

11. Dispositif selon la revendication 10, **caractérisé en ce que** l'unité de commande comporte un composant ASIC de type CJ 135.
